# EUROPEAN PATENT APPLICATION

(11) **EP 2 975 131 A1**
(43) Date of publication of application: **20.01.2016**
(21) Application number: 14177491.9
(22) Date of filing: 17.07.2014
(51) Int. Cl.: C12P 5/02, C12P 7/40, C12P 7/52

(54) **Synthesis of alkanes**

(71) Applicant: Evonik Degussa GmbH, 45128 Essen (DE)
(72) Inventor: Haas, Thomas, 48161 Münster (DE); Paulmann, Uwe, 59348 Lüdinghausen (DE); Przybilla, Simon, 48163 Münster (DE)

(57) **Abstract**

The present invention relates to a method of producing at least one alkane, the method comprising,
- producing at least one carboxylic acid from a carbon source using a genetically modified microorganism, and
- performing Kolbe electrolysis on the carboxylic acid to produce the alkane,

wherein the alkane comprises at least 6 carbon atoms and the carboxylic acid comprises at least 4 carbon atoms.

## Description

### FIELD OF THE INVENTION

The present invention related to a method of synthesising branched, unbranched and long chained alkanes from synthesis gas. In particular, the method is a biotechnological method.

### BACKGROUND OF THE INVENTION

Alkanes are saturated hydrocarbons that have various applications depending on the number of carbon atoms and on the structure of the alkane (i.e. branched, linear, cyclic etc.). The first four alkanes (CH₄ to C₄H₈) are used mainly for heating and cooking purposes and in some countries for electricity generation.

Pentane, hexane, heptane and octane are reasonably volatile liquids. They are usually used as fuels in internal combustion engines, as they vaporise easily on entry into the combustion chamber without forming droplets, which would impair the uniformity of the combustion. For this function, branched-chain alkanes are preferred as they are much less prone to premature ignition, which causes knocking, compared to their straight-chain counterparts. This propensity to premature ignition is measured by the octane rating of the fuel, where for example, 2,2,4-trimethylpentane (isooctane) has an arbitrary value of 100, and heptane has a value of zero. Apart from their use as fuels, this group of alkanes are also good solvents for nonpolar substances.

Alkanes from nonane onwards, for instance, to hexadecane (an alkane with sixteen carbon atoms) are liquids of higher viscosity, less and less suitable for use in gasoline. They form instead the major part of diesel and aviation fuel. Diesel fuels are characterized by their cetane number, cetane being an old name for hexadecane.

Alkanes from hexadecane upwards form the most important components of fuel oil and lubricating oil. In the latter function, they work at the same time as anti-corrosive agents, as their hydrophobic nature means that water cannot reach the metal surface. Many solid alkanes find use as paraffin wax, for example, in candles. This should not be confused however with true wax, which consists primarily of esters. Alkanes with a chain length of approximately 35 or more carbon atoms are found in bitumen, used, for example, in road surfacing. However, the higher alkanes have little value and are usually split into lower alkanes by cracking.

Accordingly, alkanes of all lengths are very useful in our day to day world. Currently, traditional manufacture of alkanes uses petroleum based intermediates. However, these alkanes are obtained by cracking gasoline or petroleum which is bad for the environment. Also, since the costs for these alkanes will be linked to the price of petroleum, with the expected increase in petroleum prices in the future, alkane prices may also increase relative to the increase in the petroleum prices.

Accordingly, it is desirable to find other methods of producing alkanes from more sustainable raw materials, other than purely petroleum based raw materials which also cause less damage to the environment.

There are numerous efforts underway to generate renewable fuels including alkanes from sustainable raw materials, such as synthesis gas. One method known in the art is to generate biodiesel fuel (predominantly fatty acid ethyl or methyl esters) from triglycerides. Another approach is to use the glycerol to form glycerol ethers, which can be added to biodiesel and/or diesel fuel. However, each of these methods has its' own disadvantages. There is thus still no known method of efficiently producing middle or long chained alkanes from renewable fuels including synthesis gas.

### DESCRIPTION OF THE INVENTION

The present invention provides a process of producing middle or long chained alkanes from renewable fuels. In particular, the method of the present invention may use synthesis gas to produce branched, linear and/or cyclic alkanes using at least a two-step biotechnological process. The method of the present invention may comprise at least a first step of converting synthesis gas to at least one carboxylic acid using at least a microorganism and a second step of converting the carboxylic acid to a respective alkane by subjecting the carboxylic acid to electrolysis. In particular, the electrolysis used may be Kolbe electrolysis and carbon dioxide may be produced as a bi-product.

According to one aspect of the present invention, there is provided a method of producing at least one alkane the method comprising,
- producing at least one carboxylic acid from a carbon source using a microorganism, and
- performing Kolbe electrolysis on the carboxylic acid to produce the alkane,
wherein the alkane comprises at least 6 carbon atoms and the carboxylic acid comprises at least 4 carbon atoms.

The carbon source can be in its simplest form as carbon dioxide or carbon monoxide. In particular, the carbon source may be any complex molecule with carbon in it. More in particular, the carbon source may be selected from the group consisting of alcohols, aldehydes, glucose, sucrose, fructose, dextrose, lactose, xylose, pentose, polyol, hexose, ethanol and synthesis gas. Even more in particular, the carbon source may be ethanol and/or synthesis gas. In one example, the carbon source may be ethanol in combination with at least one other carbon source selected from the group consisting of acetate, propionate, butyrate, isobutyrate, valerate and hexanoate. In particular, the carbon source may be ethanol and acetate. In another example, the carbon source may be a combination of propionic acid and ethanol, acetate and ethanol, isobutyric acid and ethanol or butyric acid and ethanol.

In one example, ethanol is the carbon source and the microorganism may be any microorganism that is capable of producing at least one carboxylic acid using the ethanol-carboxylate fermentation pathway. The ethanol-carboxylate fermentation pathway is described in detail at least in Seedorf, H., et al., 2008. The organism may be selected from the group consisting of *Clostridium kluyveri, C.Carboxidivorans* and the like. These microorganisms include microorganisms which in their wild-type form do not have an ethanol-carboxylate fermentation pathway, but have acquired this trait as a result of genetic modification. In particular, the microorganism may be *Clostridium kluyveri.*

The microorganism according to any aspect of the present invention may be a genetically modified microorganism. The genetically modified cell or microorganism may be genetically different from the wild type cell or microorganism. The genetic difference between the genetically modified microorganism according to any aspect of the present invention and the wild type microorganism may be in the presence of a complete gene, amino acid, nucleotide etc. in the genetically modified microorganism that may be absent in the wild type microorganism. In one example, the genetically modified microorganism according to any aspect of the present invention may comprise enzymes that enable the microorganism to produce at least one carboxylic acid. The wild type microorganism relative to the genetically modified microorganism of the present invention may have none or no detectable activity of the enzymes that enable the genetically modified microorganism to produce at least one carboxylic acid. As used herein, the term 'genetically modified microorganism' may be used interchangeably with the term 'genetically modified cell'. The genetic modification according to any aspect of the present invention is carried out on the cell of the microorganism.

The phrase "wild type" as used herein in conjunction with a cell or microorganism may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

A skilled person would be able to use any method known in the art to genetically modify a cell or microorganism. According to any aspect of the present invention, the genetically modified cell may be genetically modified so that in a defined time interval, within 2 hours, in particular within 8 hours or 24 hours, it forms at least twice, especially at least 10 times, at least 100 times, at least 1000 times or at least 10000 times more carboxylic acid and/or the respective carboxylic acid ester than the wild-type cell. The increase in product formation can be determined for example by cultivating the cell according to any aspect of the present invention and the wild-type cell each separately under the same conditions (same cell density, same nutrient medium, same culture conditions) for a specified time interval in a suitable nutrient medium and then determining the amount of target product (carboxylic acid) in the nutrient medium.

In one example, the microorganism may be a wild type organism that expresses at least one enzyme selected E₁ to E₁₀, wherein E₁ is an alcohol dehydrogenase (adh), E₂ is an acetaldehyde dehydrogenase (aid), E₃ is an acetoacetyl- CoA thiolase (thl), E₄ is a 3-hydroxybutyryl-CoA dehydrogenase (hbd), E₅ is a 3-hydroxybutyryl-CoA dehydratase (crt), E₆ is a butyryl-CoA dehydrogenase (bcd), E₇ is an electron transfer flavoprotein subunit (etf), E₈ is a coenzyme A transferase (cat), E₉ is an acetate kinase (ack) and E₁₀ is phosphotransacetylase (pta). In particular, the wild type microorganism according to any aspect of the present invention may express at least E₂, E₃ and E₄. Even more in particular, the wild type microorganism according to any aspect of the present invention may express at least E₄.

In another example, the microorganism according to any aspect of the present invention may be a genetically modified organism that has increased expression relative to the wild type microorganism of at least one enzyme selected E₁ to E₁₀, wherein E₁ is an alcohol dehydrogenase (adh), E₂ is an acetaldehyde dehydrogenase (aid), E₃ is an acetoacetyl-CoA thiolase (thl), E₄ is a 3-hydroxybutyryl-CoA dehydrogenase (hbd), E₅ is a 3-hydroxybutyryl-CoA dehydratase (crt), E₆ is a butyryl-CoA dehydrogenase (bcd), E₇ is an electron transfer flavoprotein subunit (etf), E₈ is a coenzyme A transferase (cat), E₉ is an acetate kinase (ack) and E₁₀ is phosphotransacetylase (pta). In particular, the genetically modified microorganism according to any aspect of the present invention may express at least enzymes E₂, E₃ and E₄. Even more in particular, the genetically modified microorganism according to any aspect of the present invention may express at least E₄. The enzymes E₁ to E₁₀ may be isolated from *Clostridium kluyveri.*

The phrase "increased activity of an enzyme", as used herein is to be understood as increased intracellular activity. Basically, an increase in enzymatic activity can be achieved by increasing the copy number of the gene sequence or gene sequences that code for the enzyme, using a strong promoter or employing a gene or allele that codes for a corresponding enzyme with increased activity and optionally by combining these measures. Genetically modified cells or microorganisms used in the method according to the invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extrachromosomally replicating vector.

The cells according to any aspect of the present invention are genetically transformed according to any method known in the art. In particular, the cells may be produced according to the method disclosed in WO/2009/077461.

The phrase 'the genetically modified cell has an increased activity, in comparison with its wild type, in enzymes'as used herein refers to the activity of the respective enzyme that is increased by a factor of at least 2, in particular of at least 10, more in particular of at least 100, yet more in particular of at least 1000 and even more in particular of at least 10000.

According to any aspect of the present invention, E₁ may be an ethanol dehydrogenase. In particular, E₁ may be selected from the group consisting of alcohol dehydrogenase 1, alcohol dehydrogenase 2, alcohol dehydrogenase 3, alcohol dehydrogenase B and combinations thereof. More in particular, E₁ may comprise sequence identity of at least 50% to a polypeptide selected from the group consisting of CKL_1075, CKL_1077, CKL_1078, CKL_1067, CKL_2967, CKL_2978, CKL_3000, CKL_3425, and CKL_2065. Even more in particular, E₁ may comprise a polypeptide with sequence identity of at least 50, 60, 65, 70, 75, 80, 85, 90, 91, 94, 95, 98 or 100% to a polypeptide selected from the group consisting of CKL_1075, CKL_1077, CKL_1078 and CKL_1067.

According to any aspect of the present invention, E₂ may be an acetaldehyde dehydrogenase. In particular, E₂ may be selected from the group consisting of acetaldehyde dehydrogenase 1, alcohol dehydrogenase 2 and combinations thereof. In particular, E₂ may comprise sequence identity of at least 50% to a polypeptide selected from the group consisting of CKL_1074, CKL_1076 and the like. More in particular, E₂ may comprise a polypeptide with sequence identity of at least 50, 60, 65, 70, 75, 80, 85, 90, 91, 94, 95, 98 or 100% to a polypeptide selected from the group consisting of CKL_1074 and CKL_1076.

According to any aspect of the present invention, E₃ may be selected from the group consisting of acetoacetyl-CoA thiolase A1, acetoacetyl-CoA thiolase A2, acetoacetyl-CoA thiolase A3 and combinations thereof. In particular, E₃ may comprise sequence identity of at least 50% to a polypeptide selected from the group consisting of CKL_3696, CKL_3697, CKL_3698 and the like. More in particular, E₃ may comprise a polypeptide with sequence identity of at least 50, 60, 65, 70, 75, 80, 85, 90, 91, 94, 95, 98 or 100% to a polypeptide selected from the group consisting of CKL_3696, CKL_3697 and CKL_3698.

According to any aspect of the present invention, E₄ may be 3-hydroxybutyryl-CoA dehydrogenase 1, 3-hydroxybutyryl-CoA dehydrogenase 2 and the like. In particular, E₄ may comprise sequence identity of at least 50% to a polypeptide CKL_0458, CKL_2795 and the like. More in particular, E₄ may comprise a polypeptide with sequence identity of at least 50, 60, 65, 70, 75, 80, 85, 90, 91, 94, 95, 98 or 100% to the polypeptide CKL_0458 or CKL_2795.

According to any aspect of the present invention, E₅ may be 3-hydroxybutyryl-CoA dehydratase 1, 3-hydroxybutyryl-CoA dehydratase 2 and combinations thereof. In particular, E₅ may comprise sequence identity of at least 50% to a polypeptide selected from the group consisting of CKL_0454, CKL_2527 and the like. More in particular, E₅ may comprise a polypeptide with sequence identity of at least 50, 60, 65, 70, 75, 80, 85, 90, 91, 94, 95, 98 or 100% to a polypeptide selected from the group consisting of CKL_0454 and CKL_2527.

According to any aspect of the present invention, E₆ may be selected from the group consisting of butyryl-CoA dehydrogenase 1, butyryl-CoA dehydrogenase 2 and the like. In particular, E₆ may comprise sequence identity of at least 50% to a polypeptide selected from the group consisting of CKL_0455, CKL_0633 and the like. More in particular, E₆ may comprise a polypeptide with sequence identity of at least 50, 60, 65, 70, 75, 80, 85, 90, 91, 94, 95, 98 or 100% to a polypeptide selected from the group consisting of CKL_0455 and CKL_0633.

According to any aspect of the present invention, E₇ may be selected from the group consisting of electron transfer flavoprotein alpha subunit 1, electron transfer flavoprotein alpha subunit 2, electron transfer flavoprotein beta subunit 1 and electron transfer flavoprotein beta subunit 2. In particular, E₇ may comprise sequence identity of at least 50% to a polypeptide selected from the group consisting of CKL_3516, CKL_3517, CKL_0456, CKL_0457 and the like. More in particular, E₇ may comprise a polypeptide with sequence identity of at least 50, 60, 65, 70, 75, 80, 85, 90, 91, 94, 95, 98 or 100% to a polypeptide selected from the group consisting of CKL_3516, CKL_3517, CKL_0456 and CKL_0457.

According to any aspect of the present invention, E₈ may be coenzyme transferase (cat). In particular, E₈ may be selected from the group consisting of butyryl-CoA: acetate CoA transferase, succinyl-CoA:coenzyme A transferase, 4-hydroxybutyryl-CoA: coenzyme A transferase and the like. More in particular, E₈ may comprise sequence identity of at least 50% to a polypeptide selected from the group consisting of CKL_3595, CKL_3016, CKL_3018 and the like. More in particular, E₈ may comprise a polypeptide with sequence identity of at least 50, 60, 65, 70, 75, 80, 85, 90, 91, 94, 95, 98 or 100% to a polypeptide selected from the group consisting of CKL_3595, CKL_3016 and CKL_3018.

According to any aspect of the present invention, E₉ may be an acetate kinase A (ack A). In particular, E₉ may comprise sequence identity of at least 50% to a polypeptide sequence of CKL_1391 and the like. More in particular, E₉ may comprise a polypeptide with sequence identity of at least 50, 60, 65, 70, 75, 80, 85, 90, 91, 94, 95, 98 or 100% to a polypeptide of CKL_1391.

According to any aspect of the present invention, E₁₀ may be phosphotransacetylase (pta). In particular, E₁₀ may comprise sequence identity of at least 50% to a polypeptide sequence of CKL_1390 and the like. More in particular, E₁₀ may comprise a polypeptide with sequence identity of at least 50, 60, 65, 70, 75, 80, 85, 90, 91, 94, 95, 98 or 100% to a polypeptide of CKL_1390.

In one example the microorganism, wild-type or genetically modified expresses E₁-E₁₀. In particular, the microorganism according to any aspect of the present invention may have increased expression relative to the wild type microorganism of E₁, E₂, E₃, E₄, E₅, E₆, E₇, E₈, E₉, E₁₀ or combinations thereof. In one example, the genetically modified microorganism has increased expression relative to the wild type microorganism of E₁, E₂, E₃, E₄, E₅, E₆, E₇, E₈, E₉ and E₁₀. More in particular, a combination of any of the enzymes E₁-E₁₀ may be present in the organism to enable at least one carboxylic acid to be produced. In one example, the genetically modified organism used according to any aspect of the present invention may comprise a combination of any of the enzymes E₁-E₁₀ that enable the organism to produce at least one, or two or three types of carboxylic acids at the same time. For example, the microorganism may be able to produce hexanoic acid, butyric acid and/or acetic acid at the simultaneously. Similarly, the microorganism may be genetically modified to express a combination of enzymes E₁-E₁₀ that enable the organism to produce either a single type of carboxylic acid or a variety of carboxylic acids. In all the above cases, the microorganism may be in its wild-type form or be genetically modified.

In one example, the genetically modified microorganism according to any aspect of the present invention has increased expression relative to the wild type microorganism of hydrogenase maturation protein and/or electron transport complex protein. In particular, the hydrogenase maturation protein (hyd) may be selected from the group consisting of hydE, hydF or hydG. In particular, the hyd may comprise sequence identity of at least 50% to a polypeptide selected from the group consisting of CKL_0605, CKL_2330, CKL_3829 and the like. More in particular, the hyd used according to any aspect of the present invention may comprise a polypeptide with sequence identity of at least 50, 60, 65, 70, 75, 80, 85, 90, 91, 94, 95, 98 or 100% to a polypeptide selected from the group consisting of CKL_0605, CKL_2330 and CKL_3829.

In one example, the microorganism according to any aspect of the present invention may be capable of producing at least valeric acid and/or heptanoic acid from a carbon source of propionic acid and ethanol. In another example, the microorganism according to any aspect of the present invention may be capable of producing at least butyric acid from a carbon source of acetate and ethanol. In yet another example, the microorganism according to any aspect of the present invention may be capable of producing at least isohexanoic acid from a carbon source of isobutyric acid and ethanol. In one further example, the microorganism according to any aspect of the present invention may be capable of producing at least hexanoic acid from a carbon source of butyric acid and ethanol or a carbon source of acetate and ethanol.

Throughout this application, any data base code, unless specified to the contrary, refers to a sequence available from the NCBI data bases, more specifically the version online on 12 June 2014, and comprises, if such sequence is a nucleotide sequence, the polypeptide sequence obtained by translating the former.

In another example, the genetically modified microorganism may be capable of producing the carboxylic acid using malonyl-CoA dependent and malonyl-ACP independent acyl-CoA metabolic pathway using any carbon source. The carbon source may be any carbon source known in the art. In particular, the carbon source may be selected from the group consisting of glucose, sucrose, fructose, dextrose, lactose, xylose, pentose, polyol, hexose and synthesis gas. More in particular, the carbon source may be synthesis gas.

According to this example, the genetically modified microorganism has
- increased expression relative to the wild type microorganism of at least one enzyme selected from the group consisting of acetoacetyl-CoA synthase (E₁₁), ketoacyl-CoA synthase (or elongase) (E₁₂), ketoacyl-CoA thiolase (E₁₃), enoyl-CoA reductase (E₁₄), ketoacyl-CoA reductase (E₁₅) and 3-hydroxyacyl-CoA dehydratase (E₁₆); and
- decreased expression relative to the wild type microorganism of acetoacetyl-CoA thiolase (E₁₇).

In particular, the genetically modified microorganism comprises at least one genetic mutation that enables the microorganism to produce at least carboxylic acid. In particular, the mutation may enable the microorganism to produce at least one carboxylic acid by means of a malonyl-CoA dependent and malonyl-ACP independent acyl-CoA metabolic pathway. More in particular, there is an increase in enzymatic activity in the malonyl-CoA dependent and malonyl-ACP independent acyl-CoA metabolic pathway in the microorganism relative to the wild type microorganism.

The microorganism may be genetically modified for increased enzymatic activity in the microorganism's malonyl-CoA dependent, malonyl-ACP independent, acyl-CoA metabolic pathway. This pathway is also referred to herein as malonyl-CoA dependent, but malonyl-ACP independent, acyl-CoA metabolic pathway. Such increase in the microorganism's malonyl-CoA dependent, malonyl-ACP independent acyl-CoA metabolic pathway can be achieved by an increased activity or expression of a gene or a pathway comprising an acetoacetyl-CoA synthase, a ketoacyl-CoA synthase (or elongase), an enoyl-CoA reductase, a ketoacyl-CoA reductase and/or a 3-hydroxyacyl-CoA dehydratase in combination with a decrease in expression or activity of acetoacetyl-CoA thiolase. Alternatively, increased activity in the microorganism's malonyl-CoA dependent, malonyl-ACP independent acyl-CoA metabolic pathway can be achieved by an increased expression of a gene or a pathway comprising an acetoacetyl-CoA synthase, a ketoacyl-CoA thiolase, a enoyl-CoA reductase, a ketoacyl-CoA reductase and/or a 3-hydroxyacyl-CoA dehydratase in combination with a decrease in expression or activity of acetoacetyl-CoA thiolase.

A list of non-limiting genetic modifications to enzymes or enzymatic activities that may lead a microorganism to produce a carboxylic acid according to any aspect of the present invention are provided in Table 2 of US20140051136. In particular, the carboxylic acid biosynthetic pathways in the microorganisms of the present invention use precursors acetyl-CoA and malonyl-CoA.

In one example, nucleic acid sequences that encode temperature-sensitive forms of these polypeptides may be introduced in place of the native enzymes, and when such genetically modified microorganisms are cultured at elevated temperatures (at which these thermolabile polypeptides become inactivated, partially or completely, due to alterations in protein structure or complete denaturation), there is observed an increase in a chemical product. For example, in *E. coli,* these temperature-sensitive mutant genes could include fabI^{ts}(S241F), fabB^{ts}(A329V) or fabD^{st}(W257Q) amongst others. In most of these examples, the genetic modifications may increase malonyl-CoA utilization so that there is a reduced conversion of malonyl-CoA to carboxylic acids via the native pathway, overall biomass, and proportionally greater conversion of carbon source to a chemical product including a carboxylic acid or carboxylic acid derived product via a malonyl-CoA dependent and malonyl-ACP independent route. Also, additional genetic modifications, such as to increase malonyl-CoA production, may be made for some examples.

In another example, the enzyme, enoyl-acyl carrier protein reductase (EC No. 1.3.1.9, also referred to as enoyl-ACP reductase) is a key enzyme for carboxylic acid biosynthesis from malonyl-CoA. In *Escherichia coli* this enzyme, Fabl, is encoded by the gene fabl (Richard J. Heath et al., 1995).

In one example, the expression levels of a pyruvate oxidase gene (Chang et al., 1983, Abdel-Ahmid et al., 2001) can be reduced or functionally deleted in the microorganism according to any aspect of the present invention. The pyruvate oxidase gene may encode an enzyme of, for example, EC 1.2.3.3. In particular, the pyruvate oxidase gene may be a poxB gene.

In one example, the expression levels of a lactate dehydrogenase gene (Mat-Jan et al., Bunch et al., 1997) can be reduced or functionally deleted. In some examples, the lactate dehydrogenase gene encodes an enzyme of, for example, EC 1.1.1.27. The lactate dehydrogenase gene may be an NAD-linked fermentative D-lactate dehydrogenase gene. In particular, the lactate dehydrogenase gene is a IdhA gene

In one example, the genetic mutation may be in at least one feedback resistant enzyme of the microorganism that results in increased expression of the feedback resistant enzyme. In particular, the enzyme may be pantothenate kinase, pyruvate dehydrogenase or the like. In *E. coli,* these feedback resistant mutant genes could include coaA(R106A) and/or Ipd(E354K).

In a further example, the increase in the microorganism's malonyl-CoA dependent, but malonyl-ACP independent acyl-CoA metabolic pathway may occur through reduction in the acetoacetyl-CoA thiolase activity and/or trigger factor activity and/or in the activity of a molecular chaperone involved in cell division. In one example, the cell may comprise a genetic mutation in tig gene.

In one example, the genetic mutation in the microorganism may result in increased enzymatic activity in the NADPH-dependent transhydrogenase pathway relative to the wild type cell. This result may occur by introduction of a heterologous nucleic acid sequence coding for a polypeptide encoding nucleotide transhydrogenase activity.

In another example, the genetic mutation in the microorganism may result in decreased expression of carboxylic acyl-CoA synthetase and/or ligase activity via any method known in the art.

In yet another example, the genetic mutation in the microorganism may result in overexpression of an enzyme having acetyl-CoA carboxylase activity.

In one example, the microorganism may have increased intracellular bicarbonate levels brought about by introduction of a heterologous nucleic acid sequence coding for a polypeptide having cyanase and/or carbonic anhydrase activity.

More in particular, the genetic mutation according to any aspect of the microorganism may result in increased and/ or decreased levels of acyl-CoA thioesterase activity. This result may increase chain length specificity of a desired carboxylic acid product by increasing levels of chain length specific acyl-CoA thioesterase activity and decreasing the activity of acyl-CoA thioesterase activity on undesired carboxylic acid chain lengths. In one example, the increased chain length specificity of carboxylic acid or carboxylic acid derived product may occur by increasing levels of chain length specific ketoacyl-CoA thiolase, enoyl-CoA reductase, ketoacyl-CoA reductase or 3-hydroxyacyl-CoA dehydratase activities either individually or in combination.

The genetic mutation in the microorganism according to any aspect of the present invention may result in an increase or decrease in expression of only one enzyme selected from the list of enzymes mentioned above or an increase or decrease in expression of a combination of enzymes mentioned above.

In another example, the genetic mutation in the microorganism may be in at least one enzyme selected from the group consisting of acetoacetyl-CoA synthase, ketoacyl-CoA synthase (or elongase), enoyl-CoA reductase, ketoacyl-CoA reductase, 3-hydroxyacyl-CoA dehydratase and acetoacetyl-CoA thiolase. More in particular, the genetic mutation in the microorganism may result in an increase in expression of acetoacetyl-CoA synthase, ketoacyl-CoA synthase (or elongase), enoyl-CoA reductase, ketoacyl-CoA reductase and 3-hydroxyacyl-CoA dehydratase in combination with a decrease in expression or activity of acetoacetyl-CoA thiolase. In particular, the enoyl-CoA reductase and/or ketoacyl-CoA reductase may either utilize the cofactor NADH and/or NADPH.

In particular, the genetic modification in the microorganism according to any aspect of the present invention may comprise any of the enzymes listed in Table 1 in combination with the following enzymes acetoacetyl-CoA synthase, ketoacyl-CoA synthase (or elongase), enoyl-CoA reductase, ketoacyl-CoA reductase and/or 3-hydroxyacyl-CoA dehydratase and acetoacetyl-CoA thiolase wherein the expression or activity of enzymes acetoacetyl-CoA synthase, ketoacyl-CoA synthase (or elongase), enoyl-CoA reductase, ketoacyl-CoA reductase and 3-hydroxyacyl-CoA dehydratase is increased and the activity of acetoacetyl-CoA thiolase is decreased.

In yet another example, malonyl-CoA dependent, malonyl-ACP independent acyl-CoA metabolic pathway in the microorganism according to any aspect of the present invention can be achieved by an increased expression of a gene or a pathway comprising acetoacetyl-CoA synthase, ketoacyl-CoA thiolase, enoyl-CoA reductase, ketoacyl-CoA reductase and/or 3-hydroxyacyl-CoA dehydratase in combination with a decrease in expression or activity of acetoacetyl-CoA thiolase.

In particular, the genetic modification in the microorganism according to any aspect of the present invention may comprise any of the enzymes listed in Table 2 of US20140051136 in combination with the following enzymes acetoacetyl-CoA synthase, ketoacyl-CoA thiolase, enoyl-CoA reductase, ketoacyl-CoA reductase and/or 3-hydroxyacyl-CoA dehydratase in combination with a decrease in expression or activity of acetoacetyl-CoA thiolase.

In one example, the microorganism according to any aspect of the present invention may comprise a genetic modification in any of the enzymes listed in Table 1 in combination with the following enzymes acetyl-CoA carboxylase, malonyl-CoA:ACP transacylase (FabD), β-ketoacyl-ACP synthase III, β-ketoacyl-ACP synthase I (FabB), β-ketoacyl-ACP synthase II (FabF), 3-oxoacyl-ACP-synthase I and enoyl ACP reductase.

More in particular, the genetic mutation may result in an increase in the expression of at least one enzyme selected from the group consisting of acetyl-CoA carboxylase, malonyl-CoA:ACP transacylase (FabD), β-ketoacyl-ACP synthase III, β-ketoacyl-ACP synthase I (FabB), β-ketoacyl-ACP synthase II (FabF), 3-oxoacyl-ACP-synthase I and enoyl ACP reductase relative to the wild type microorganism. In particular, the genetic mutation may result in an increase in the expression of more than one enzyme in the microorganism according to any aspect of the present invention that enables the microorganism to produce a carboxylic acid by means of increased enzymatic activity in the microorganism relative to the wild type microorganism of malonyl-CoA dependent and malonyl-ACP independent acyl-CoA metabolic pathway.

In one example, there may be an increase in expression of β-ketoacyl-ACP synthase and 3-oxoacyl-ACP-synthase in the microorganism according to any aspect of the present invention. In another example, there may be an increase in expression of β-ketoacyl-ACP synthase and Malonyl-CoA-ACP transacylase in the microorganism according to any aspect of the present invention. In yet another example, there may be an increase in expression of β-ketoacyl-ACP synthase and enoyl ACP reductase in the microorganism according to any aspect of the present invention. In one example, there may be an increase in expression of β-ketoacyl-ACP synthase, Malonyl-CoA-ACP transacylase and enoyl ACP reductase in the microorganism according to any aspect of the present invention. In all examples, there may be an increase in the expression of enoyl ACP reductase and/or acyl-CoA thioesterase.

According to any aspect of the present invention, the genetically modified organism may at least be one acetogenic bacteria and/or hydrogen oxidising bacteria capable of producing at least one carboxylic acid from a carbon source. The carbon source may be synthesis gas or any carbohydrate known in the art. In particular, the carbon source may be selected from the group consisting of alcohols, aldehydes, glucose, sucrose, fructose, dextrose, lactose, xylose, pentose, polyol, hexose, ethanol and synthesis gas. Even more in particular, the carbon source may be synthesis gas.

Usually, a portion of the synthesis gas obtained from the gasification process is first processed in order to optimize product yields, and to avoid formation of tar. Cracking of the undesired tar and CO in the synthesis gas may be carried out using lime and/or dolomite. These processes are described in detail in for example, Reed, 1981.

The synthesis gas may be converted to a carboxylic acid in the presence of at least one acetogenic bacteria and/or hydrogen oxidising bacteria. In particular, any method known in the art may be used. The carboxylic acid may be produced from synthesis gas by at least one prokaryote. In particular, the prokaryote may be selected from the group consisting of the genus *Escherichia* such as *Escherichia coli;* from the genus *Clostridia* such as *Clostridium Ijungdahlii, Clostridium autoethanogenum, Clostridium carboxidivorans* or *Clostridium kluyveri;* from the genus *Corynebacteria* such as *Corynebacterium glutamicum;* from the genus *Cupriavidus* such as *Cupriavidus necator* or *Cupriavidus metallidurans;* from the genus *Pseudomonas* such as *Pseudomonas fluorescens, Pseudomonas putida* or *Pseudomonas oleavorans;* from the genus *Delftia* such as *Delftia acidovorans;* from the genus *Bacillus* such as *Bacillus subtillis*; from the genus *Lactobacillus* such as *Lactobacillus delbrueckii;* or from the genus *Lactococcus* such as *Lactococcus lactis.*

In another example, carboxylic acid may be produced from synthesis gas by at least one eukaryote. The eukaryote used in any aspect of the present invention may be selected from the genus *Aspergillus* such as *Aspergillus niger;* from the genus *Saccharomyces* such as *Saccharomyces cerevisiae;* from the genus *Pichia* such as *Pichia pastoris;* from the genus *Yarrowia* such as *Yarrowia lipolytica;* from the genus *Issatchenkia* such as *Issathenkia orientalis;* from the genus *Debaryomyces* such as *Debaryomyces hansenii;* from the genus *Arxula* such as *Arxula adenoinivorans*; or from the genus *Kluyveromyces* such as *Kluyveromyces lactis.*

The carboxylic acid may be any carboxylic known in the art. In particular, the carboxylic acid may be selected from the group consisting of Butanoic acid (CH₃(CH₂)₂COOH), Pentanoic acid (CH₃(CH₂)₃COOH), Hexanoic acid (CH₃(CH₂)₄COOH), Heptanoic acid (CH₃(CH₂)₅COOH, and Octanoic acid (CH₃(CH₂)₆COOH). In one example, the carboxylic acid may be selected from the group consisting of butyric, valeric, isovaleric, hexanoic, isohexanoic, heptanoic, isoheptanoic, octanoic and isooctanoic acid.

In one example, the carboxylic acid may be hexanoic acid. In particular, the hexanoic acid may be produced from synthesis gas by any method disclosed in Steinbusch, 2011, Zhang, 2013, Van Eerten-Jansen, M. C. A. A, 2013, Ding H. et al, 2010, Barker H.A., 1949, Stadtman E.R., 1950, Bornstein B. T., et al., 1948 and the like. Even more in particular, the hexanoic acid may be produced from synthesis gas in the presence of at least *Clostridium kluyveri.*

The term "acetogenic bacteria" as used herein refers to a microorganism which is able to perform the Wood-Ljungdahl pathway and thus is able to convert CO, CO₂ and/or hydrogen to acetate. These microorganisms include microorganisms which in their wild-type form do not have a Wood-Ljungdahl pathway, but have acquired this trait as a result of genetic modification. Such microorganisms include but are not limited to *E. coli* cells. Currently, 21 different genera of the acetogenic bacteria are known in the art (Drake et al., 2006), and these may also include some *clostridia* (Drake & Kusel, 2005). These bacteria are able to use carbon dioxide or carbon monoxide as a carbon source with hydrogen as an energy source (Wood, 1991). Further, alcohols, aldehydes, carboxylic acids as well as numerous hexoses may also be used as a carbon source (Drake et al., 2004). The reductive pathway that leads to the formation of acetate is referred to as acetyl-CoA or Wood-Ljungdahl pathway.

In particular, the acetogenic bacteria may be selected from the group consisting of *Clostridium autothenogenum* DSMZ 19630, *Clostridium ragsdahlei ATCC* no. BAA-622, *Clostridium autoethanogenum, Clostridium carboxidivorans* DSM 15243, *Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum* ATCC 33266, *Clostridium formicoaceticum, Clostridium butyricum, Laktobacillus delbrukii, Propionibacterium acidoprprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium Ijungdahlii, Clostridium* ATCC 29797 and *Clostridium carboxidivorans.* More in particular, the strain ATCC BAA-624 of *Clostridium carboxidivorans* may be used. Even more in particular, the bacterial strain labelled "P7" and "P11" of *Clostridium carboxidivorans* as described for example in U.S. 2007/0275447 and U.S. 2008/0057554 may be used.

Another particularly suitable bacterium may be *Clostridium Ijungdahlii.* In one example, in the production of hexanoic acid from synthesis gas, strains selected from the group consisting of *Clostridium Ijungdahlii* PETC, *Clostridium Ijungdahlii* ERI2, *Clostridium Ijungdahlii* COL and *Clostridium Ijungdahlii* O-52 may be used. These strains for example are described in WO 98/00558, WO 00/68407, ATCC 49587, ATCC 55988 and ATCC 55989.

The acetogenic bacteria may be used in conjunction with a hydrogen oxidising bacteria. In one example, both an acetogenic bacteria and a hydrogen oxidising bacteria may be used to produce carboxylic acid from synthesis gas. In another example, only acetogenic bacteria may be used for metabolising synthesis gas to produce carboxylic acid from synthesis gas. In yet another example, only a hydrogen oxidising bacteria may be used in this reaction.

The hydrogen oxidising bacteria may be selected from the group consisting of *Achromobacter, Acidithiobacillus, Acidovorax, Alcaligenes, Anabena, Aquifex, Arthrobacter, Azospirillum, Bacillus, Bradyrhizobium, Cupriavidus, Derxia, Helicobacter, Herbaspirillum, Hydrogenobacter, Hydrogenobaculum, Hydrogenophaga" Hydrogenophilus, Hydrogenothermus, Hydrogenovibrio, Ideonella sp.* O1, *Kyrpidia, Metallosphaera, Methanobrevibacter, Myobacterium, Nocardia, Oligotropha, Para coccus, Pelomonas, Polaromonas, Pseudomonas, Pseudonocardia, Rhizobium, Rhodococcus, Rhodopseudomonas, Rhodospirillum, Streptomyces, Thiocapsa, Treponema,Variovorax, Xanthobacter* and *Wa utersia.*

In the production of carboxylic acid from synthesis gas a combination of bacteria may be used. There may be more than one acetogenic bacteria present in combination with one or more hydrogen oxidising bacteria. In another example, there may be more than one type of acetogenic bacteria present only. In yet another example, there may be more than one hydrogen oxidising bacterium present only.

The method according to any aspect of the present invention may comprise a step of extracting the carboxylic acid produced from the synthesis gas first before performing electrolysis. Any method known in the art for extracting carboxylic acid may be used. In particular, one example of an extraction method of carboxylic acid is provided in section 2.3 of Byoung, S.J et al. 2013. Another example may the method disclosed under the section 'Extraction Model' in Kieun C., et al., 2013.

The carboxylic acid may then be subjected to Kolbe electrolysis, or to photo-Kolbe conditions. This reaction may result in the formation of the respective alkane, carbon dioxide and hydrogen. The alkanes produced may be more immiscible. This allows for the alkane produced according to any aspect of the present invention to be easily separated. The method according to any aspect of the present invention may be considered to be highly selective and may allow for alkanes produced to have high purity. In one example, when hexanoic acid may be subjected to Kolbe electrolysis a decane (C₁₀H₂₂) and hydrogen, with minor amounts of pentane are formed. The hexane may be immiscible with the fermentation broth, and may be easily separated.

Kolbe electrolysis conditions are known in the art, and include platinum electrodes, sonoemulsion with various electrodes such as boron-doped chemical vapour deposition diamond electrodes (Wadhawan J.D., et al., 2001), polymer electrodes, and the like. Kolbe electrolysis synonymous to Kolbe fatty acid or carboxylic acid electrolysis is a means of oxidative decarboxylation of carboxylic acids. Kolbe electrolysis typically works with the carboxylate anion rather than the acid itself.

In particular, the Kolbe electrolysis is an example of an organic redox reaction that takes place in an electrochemical cell. This method has several advantages which include for example the possibility to control the potential of the electrode. It may also be considered a simple reaction because no reducing or oxidizing agents are required. In Kolbe electrolysis, the oxidation does not take place chemically but electrolytically. Alkanes are formed by dimerisation of the generated radicals according to the reaction shown below.

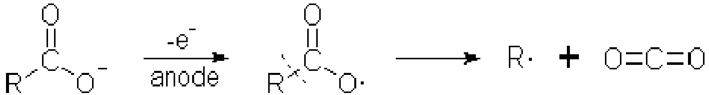

R· + R· → R-R

Kolbe electrolysis may also be known to produce side products which depend on the ease of the follow-up oxidation which leads to carbenium ions and their subsequent rearrangement:

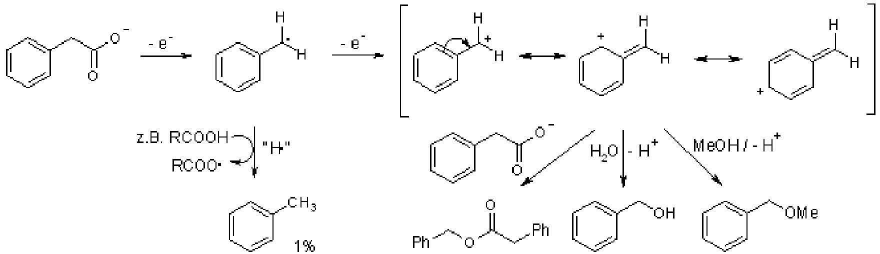

In one example, the Kolbe electrolysis is carried out on the fermentation broth itself that was used for carboxylic acid production in step (i) of the method according to any aspect of the present invention. This allows the alkane product to separate from the fermentation broth. The alkane may then be easily separated by decantation, distillation or other means known in the art. Thus, a continuous or semi-continuous process can be used, where synthesis gas is added to the system while the alkane is formed, and the alkane may be removed, for example, using evaporative distillation, decantation, and the like from the fermentation broth. Both steps of (i) carboxylic acid production by the acetogenic bacteria and/or hydrogen oxidising bacteria and (ii) Kolbe electrolysis on the produced carboxylic acid may be carried out in a single fermenter and both steps may be carried out simultaneously. No separation of the carboxylic acid between steps (i) and (ii) may be needed.

According to any aspect of the present invention, the Kolbe electrolysis may comprise the presence of a salt, usually the alkali salt of the carboxylic acid, water and two metal electrodes. A current and voltage of at least 1V is available between the two electrodes.

In another example, the fermentation is first completed, and then the carboxylic acid ions may be optionally isolated, before the Kolbe electrolysis is performed. The carboxylic acid can be removed from the fermentation broth, for example, by continuous extraction with a solvent. In this case, since the Kolbe electrolysis or photo-Kolbe electrolysis occurs after the fermentation takes place, the acetogenic bacteria can be collected, for example, by decantation or filtration of the fermentation media, and a new batch of water containing synthesis gas can be combined with the acetogenic bacteria. The acetogenic bacteria may thus be recycled. The fermentation media including the carboxylic acid can be subjected to Kolbe electrolysis or photo-Kolbe electrolysis, and since the resulting products (respective alkane, bi-product alkane, hydrogen and carbon dioxide) easily separate from the aqueous fermentation solution, they can be easily isolated, for example, by collecting the gases (bi-product alkane, carbon dioxide and hydrogen) and separating them into their components, and by decanting or distilling the respective alkane. The aqueous solution can then be recycled to the fermenter, if desired, or otherwise disposed of. In this example, both steps may also be carried out in a single fermenter or separate fermenters.

The method according to any aspect of the present invention may also provide a source of hydrogen, derived from both the fermentation step and the Kolbe electrolysis step. The hydrogen produced by the process can be used in fuel cells, in a Fischer-Tropsch reactor, as a fuel, or in any other appropriate use for hydrogen. Gasoline, jet fuel, and diesel fuel derived from Fischer-Trospch synthesis are all well known to those of skill in the art. The carbon dioxide produced by the process can be trapped by algae, and used to produce triglycerides. The triglycerides can be used, for example, to produce biodiesel fuel.

In one example, one or more carboxylic acids may be subjected to the Kolbe electrolysis or photo-Kolbe electrolysis step at the same time. These carboxylic acids will form radicals of the alkyl group to which the carboxylic acid group is attached as shown above. These radicals can react with any alkyl radicals formed by the decarboxylation of carboxylic acid.

The carboxylic acid used according to any aspect of the present invention may be saturated, unsaturated and/or branched or unbranched. Accordingly, the resultant alkane may also be branched or unbranched depending on the carboxylic acid used. In particular the resultant alkane may be symmetrical or unsymmetrical. A symmetrical alkane may be an alkane in which half of the molecule is a mirror image of the other half.

In particular, according to any aspect of the present invention, the alkane and carboxylic acid are selected from the group consisting of:
(a) the alkane comprising 6 carbon atoms and the carboxylic acid comprising 4 carbon atoms;
(b) the alkane comprising 8 carbon atoms and the carboxylic acid comprising 5 carbon atoms;
(c) the alkane comprising 8 carbon atoms and a first carboxylic acid comprising 6 carbon atoms and a second carboxylic acid comprising 4 carbon atoms;
(d) the alkane comprising 10 carbon atoms and the carboxylic acid comprising 6 carbon atoms;
(e) the alkane comprising 10 carbon atoms and a first carboxylic acid comprising 5 carbon atoms and a second carboxylic acid comprising 7 carbon atoms;
(f) the alkane comprising 12 carbon atoms and the carboxylic acid comprising 7 carbon atoms;
(g) the alkane comprising 12 carbon atoms and a first carboxylic acid comprising 8 carbon atoms and a second carboxylic acid comprising 6 carbon atoms;
(h) the alkane comprising 12 carbon atoms and a first carboxylic acid comprising 9 carbon atoms and a second carboxylic acid comprising 5 carbon atoms;
(i) the alkane comprising 14 carbon atoms and the carboxylic acid comprising 8 carbon atoms; and
(j) the alkane comprising 14 carbon atoms and a first carboxylic acid comprising 6 carbon atoms and a second carboxylic acid comprising 8 carbon atoms.;
(k) alkane comprising 16 carbon atoms and the carboxylic acid comprising 9 carbon atoms;
(I) alkane comprising 9 carbon atoms and a first carboxylic acid comprising 5 carbon atoms and a second carboxylic acid comprising 6 carbon atoms;
(m) alkane comprising 11 carbon atoms and a first carboxylic acid comprising 6 carbon atoms and a second carboxylic acid comprising 7 carbon atoms; and
(n) alkane comprising 13 carbon atoms and a first carboxylic acid comprising 7 carbon atoms and a second carboxylic acid comprising 8 carbon atoms.; and
(o) alkane comprising 15 carbon atoms and a first carboxylic acid comprising 7 carbon atoms and a second carboxylic acid comprising 8 carbon atoms.

More in particular, the alkane and carboxylic acid are selected from the group consisting of:
(a) the alkane comprising 10 carbon atoms and the carboxylic acid comprising 6 carbon atoms;
(b) the alkane comprising 12 carbon atoms and the carboxylic acid comprising 7 carbon atoms; and
(c) the alkane comprising 14 carbon atoms and the carboxylic acid comprising 8 carbon atoms;

In one example, the carboxylic acid used in any aspect of the present invention may comprise at least 4 carbon atoms (butanoic acid/butyric acid). When butyric acid is subjected to Kolbe electrolysis, the resultant alkane may comprise at least 6 carbon atoms. In particular, the resultant alkane may be a hexane. In another example, when a combination of carboxylic acids comprising at least a first carboxylic acid of butyric acid and a second carboxylic acid of propionic acid (3 carbon atoms) the resultant alkane may comprise at least 5 carbon atoms. In particular, the resultant alkane may be a pentane. Bi-products such as hexane and butane may also be formed in this example.

In one example, the carboxylic acid used in any aspect of the present invention may comprise at least 5 carbon atoms (pentanoic acid/ valeric acid). When pentanoic acid is subjected to Kolbe electrolysis, the resultant alkane may comprise at least 8 carbon atoms. In particular, the resultant alkane may be an octane. In a further example, when a combination of carboxylic acids comprising at least a first carboxylic acid of pentanoic acid and a second carboxylic acid of propionic acid the resultant alkane may comprise at least 6 carbon atoms. In particular, the resultant alkane may be a hexane. Bi-products such as octane and butane may also be formed in this example. In yet another example, when a combination of carboxylic acids comprising at least a first carboxylic acid of pentanoic acid and a second carboxylic acid of butanoic acid the resultant alkane may comprise at least 7 carbon atoms. In particular, the resultant alkane may be a heptane. Bi-products such as octane and hexane may also be formed in this example.

In one example, the carboxylic acid used in any aspect of the present invention may comprise at least 6 carbon atoms (hexanoic acid/ caproic acid). When hexanoic acid is subjected to Kolbe electrolysis, the resultant alkane may comprise at least 10 carbon atoms. In particular, the resultant alkane may be a decane. In another example, when a combination of carboxylic acids comprising at least a first carboxylic acid of hexanoic acid and a second carboxylic acid of propionic acid the resultant alkane may comprise at least 7 carbon atoms. In particular, the resultant alkane may be a heptane. Bi-products such as decane and butane may also be formed in this example. In another example, when a combination of carboxylic acids comprising at least a first carboxylic acid of hexanoic acid and a second carboxylic acid of butanoic acid the resultant alkane may comprise at least 8 carbon atoms. In particular, the resultant alkane may be an octane. Bi-products such as decane and hexane may also be formed in this example. In yet another example, when a combination of carboxylic acids comprising at least a first carboxylic acid of hexanoic acid and a second carboxylic acid of pentanoic acid, the resultant alkane may comprise at least 9 carbon atoms. In particular, the resultant alkane may be a nonane. Bi-products such as decane and octane may also be formed in this example.

In one example, the carboxylic acid used in any aspect of the present invention may comprise at least 7 carbon atoms (heptanoic acid/ enanthic acid). When heptanoic acid is subjected to Kolbe electrolysis, the resultant alkane may comprise at least 12 carbon atoms. In particular, the resultant alkane may be a dodecane. In another example, when a combination of carboxylic acids comprising at least a first carboxylic acid of heptanoic acid and a second carboxylic acid of propionic acid the resultant alkane may comprise at least 8 carbon atoms. In particular, the resultant alkane may be an octane. Bi-products such as dodecane and butane may also be formed in this example. In another example, when a combination of carboxylic acids comprising at least a first carboxylic acid of heptanoic acid and a second carboxylic acid of butanoic acid the resultant alkane may comprise at least 9 carbon atoms. In particular, the resultant alkane may be a nonane. Bi-products such as dodecane and hexane may also be formed in this example. In yet another example, when a combination of carboxylic acids comprising at least a first carboxylic acid of heptanoic acid and a second carboxylic acid of pentanoic acid, the resultant alkane may comprise at least 10 carbon atoms. In particular, the resultant alkane may be a decane. Bi-products such as dodecane and octane may also be formed in this example. In a further example, when a combination of carboxylic acids comprising at least a first carboxylic acid of heptanoic acid and a second carboxylic acid of hexanoic acid, the resultant alkane may comprise at least 11 carbon atoms. In particular, the resultant alkane may be an undecane. Bi-products such as dodecane and decane may also be formed in this example.

In one example, the carboxylic acid used in any aspect of the present invention may comprise at least 8 carbon atoms (octanoic acid/ caprylic acid). When octanoic acid is subjected to Kolbe electrolysis, the resultant alkane may comprise at least 14 carbon atoms. In particular, the resultant alkane may be a tetradecane. In another example, when a combination of carboxylic acids comprising at least a first carboxylic acid of octanoic acid and a second carboxylic acid of propionic acid the resultant alkane may comprise at least 9 carbon atoms. In particular, the resultant alkane may be a nonane. Bi-products such as tetradecane and butane may also be formed in this example. In another example, when a combination of carboxylic acids comprising at least a first carboxylic acid of octanoic acid and a second carboxylic acid of butanoic acid the resultant alkane may comprise at least 10 carbon atoms. In particular, the resultant alkane may be a decane. Bi-products such as tetradecane and hexane may also be formed in this example. In yet another example, when a combination of carboxylic acids comprising at least a first carboxylic acid of octanoic acid and a second carboxylic acid of pentanoic acid, the resultant alkane may comprise at least 11 carbon atoms. In particular, the resultant alkane may be an undecane. Bi-products such as tetradecane and octane may also be formed in this example. In a further example, when a combination of carboxylic acids comprising at least a first carboxylic acid of octanoic acid and a second carboxylic acid of hexanoic acid, the resultant alkane may comprise at least 12 carbon atoms. In particular, the resultant alkane may be a dodecane. Bi-products such as tetradecane and decane may also be formed in this example. In yet another example, when a combination of carboxylic acids comprising at least a first carboxylic acid of octanoic acid and a second carboxylic acid of heptanoic acid, the resultant alkane may comprise at least 13 carbon atoms. In particular, the resultant alkane may be a tridecane. Bi-products such as tetradecane and dodecane may also be formed in this example.

In particular, the carboxylic acid used in any aspect of the present invention may comprise at least 6, 7, or 14 carbon atoms. When the acid is subjected to Kolbe electrolysis, the resultant alkane may comprise at least 10, 12 or 14 carbon atoms respectively. A combination of carboxylic acids may be used in any aspect of the present invention. There may be 2, 3, 4, 5, 6, 7, 8, 9, 10 or more carboxylic acids subjected to Kolbe electrolysis. A mixture of alkanes may be formed depending on which first acid dimerises with which second acid. A skilled person would be able to select the carboxylic acids to undergo Kolbe electrolysis to produce the desired alkane(s).

The method according to any aspect of the present invention is advantageous as it provides hydrocarbons in the gasoline range, from the same starting materials as those used to form ethanol, but which have higher energy per unit volume.

The microorganisms according to any aspect of the present invention may be held in immobilized cell bioreactors, such as fibrous-bed bioreactors (FBBs), to carry out the fermentation. However, any known fermenter may be used. A skilled person would easily be able to determine the most suitable fermenter to use based on the method used to obtain carboxylic acid, the type of carboxylic acids produced, the conditions for growth of the microorganisms and the like.

The use of immobilized cell bioreactors helps re-use the bacteria. However, in examples where Kolbe electrolysis and/or photo-Kolbe electrolysis is carried out on the fermentation media, the alkane will be easily removed from the reaction media, so immobilization of the bacteria will not be a problem in such cases. When the bacteria are not immobilized, they can be isolated, for example, using centrifugal bacterial reclamation.

Continuous decantation, evaporative removal, or extraction of the alkane allows the fermentation medium to be continuously sterilized and minimizes water use. The non-stop nature of the fermentation process allows substrate concentrations to be constantly kept at optimal levels and therefore fermentation efficiency is maximized.

The carboxylic acid(s) formed during the fermentation can be converted to alkane via Kolbe electrolysis. Kolbe electrolysis is an anodic oxidation process of a carboxylate anion. A radical is formed, which then decarboxylates. The resulting radical combines with another to form a dimer. For example, the carboxylic acid formed according to any aspect of the present invention may lose a mole of carbon dioxide to produce a alkyl radical, two of which will combine to form the respect alkane. The efficiency of Kolbe electrolysis is sensitive to water. Therefore, in one example, the reaction may be run in (almost) water free conditions.

In one example, the Kolbe electrolysis may be performed in an ionic liquid, which can optionally be present in the fermenter. In another example, the anion exchange membranes are used as solid polymer electrolytes (http://www.pca-gmbh.com/appli/spe.htm). One example of a suitable electrode system is a monel cathode and platinum sheet or platinum gauze anode.

In Kolbe electrolysis, there are some competing reactions, such as an elimination reaction which would convert the carboxylic acid to alkene, carbon dioxide and/or hydrogen. Low temperatures (i.e., 30-40°C) and high flow rates over the electrodes tend to favour paraffin formation, rather than elimination.

The alkane according to any aspect of the present invention may be used to produce at least one oxidised alkane product, wherein the method comprises contacting the alkane according to any aspect of the present invention with at least one second microorganism capable of oxidising the alkane to the respective oxidised alkane product, wherein the oxidised alkane product is selected from the group consisting of respective alcohols, carboxylic acids and dicarboxylic acids and the second microorganism is selected from the group consisting of *E. coli, Candida tropicalis, Yarrowia lipolytica,* and *Pseudomonas putida.*

In one example, the microorganism according to any aspect of the present invention may be further genetically modified to be capable of oxidising the alkane to any one of the respective oxidised products. In another example, the alkane produced according to any aspect of the present invention is fed to a second microorganism that may be capable of carrying out the oxidation of the alkane.

The second microorganism according to any aspect of the present invention may be selected from the group consisting of *E. coli, Candida tropicalis, Yarrowia lipolytica, Pseudomonas putida* and the like. In particular, the second microorganism may be genetically modified to increase the expression of at least one enzyme that may be capable of oxidising the alkane. In one example, the second microorganism may be genetically modified to express a recombinant alkane hydroxylase. In particular, the alkane hydroxylase may be a cytochrome P450 monooxygenase of the CYP153 family. The term "cytochrome P450 monooxygenase of the CYP153 family" may refer herein to a cytosolic oxidase which is part of a 3-component system comprising furthermore a ferredoxin and a ferredoxin reductase, with an alkane-binding site and the ability to hydroxylate alkanes. More in particular, the cytochrome P450 monooxygenase of the CYP153 family may have at least 80, 90, 95 or 99 % polypeptide sequence identity with cytochrome P450 monooxygenase of the CYP153 family from *Alcanivorax borkumensis* SK2 (database code YP_691921). The second microorganism may further have alkane hydroxylase activity.

In particular, the term "alkane hydroxylase activity", as used herein, refers to the ability to catalyse the hydroxylation of alkanes or unsubstituted linear alkyl radicals comprising at least six, more in particular at least twelve, carbon radicals. The term "cytochrome P450 monooxygenase of the CYP153 family" means a non-membrane-bound oxidase which comprises a binding site for alkanes, unsubstituted linear alkyl radicals comprising at least five, or particularly twelve, carbon radicals or monohydroxylated alkanes, and the polypeptide chain of which comprises the motif LL(I/L)(V/I)GGNDTTRN. In one example, a "cytochrome P450 monooxygenase of the CYP153 family", as used herein, is a cytochrome P450 monooxygenase of the CYP153 family from *Alcanivorax borkumensis* SK2 (database code YP_691921) or a variant which preferably has alkane hydroxylase activity.

The enzymes used according to any aspect of the present the invention may be recombinant enzymes. The term "recombinant", as used herein, refers to the possibility that the corresponding nucleic acid molecule may not be present in the natural cell and/or was produced using genetic engineering methods. In one example, a protein is said to be recombinant if the corresponding polypeptide is encoded by a recombinant nucleic acid. Similarly, a recombinant cell, as used herein, refers to a cell which has at least one recombinant nucleic acid or one recombinant polypeptide. A person skilled in the art is familiar with processes suitable for producing recombinant molecules or cells. Recombinant enzymes may be overexpressed, for example by using pET- or pGEX-vector systems which are known to a person skilled in the art.

In one example, to supply cytochrome P450 monooxygenase of the CYP153 family with electrons from the reducing agent, preferably NADH, in an optimal way, the cell expressing the monooxygenase together with ferredoxin reductase and ferredoxin, both of which interact functionally with said monooxygenase may be used. The polypeptides may be isolated or, when using a whole cell catalyst, co-expressed polypeptides or polypeptides fused N- or C-terminally to the cytochrome P450 monooxygenase of the CYP153 family. A person skilled in the art can readily determine whether a ferredoxin reductase or a ferredoxin interacts functionally with a given cytochrome P450 monooxygenase of the CYP153 family by whether the reducing agent is oxidized in the presence of an alkane substrate and the three polypeptides. Alternatively, the enzyme assay described by Scheps, D. et al. (2011) may be used, which shows a distinct increase in the reaction rate in the case of functionally interacting polypeptides. In one example, cytochrome P450 monooxygenase of the CYP153 family, ferredoxin and ferredoxin reductase are from the same organism. In another example, ferredoxin reductase may be that from *Alcanivorax borkumensis* SK2 (database code YP_691923) or may be a variant thereof, ferredoxin may be that from *Alcanivorax borkumensis* SK2 (database code YP_691920) or is a variant thereof, and cytochrome P450 monooxygenase of the CYP153 family may be that from *Alcanivorax borkumensis* SK2 (database code YP_691921) or is a variant thereof.

In another example, the alkane hydroxylase may be an AlkB monooxygenase. AlkB is an oxidoreductase first known from the *Pseudomonas putida* Gpo1 AlkBGT system, which is dependent on another two polypeptides, AlkG and AlkT. AlkT is characterized as FAD-dependent rubredoxin reductase that passes on electrons from NADH to AlkG. AlkG is a rubredoxin, an iron-containing redox protein that acts as a direct electron donor for AlkB. The term "AlkB monooxygenase" used herein may refer to a polypeptide with a sequence homology of at least, in the order of increasing preference, 75, 80, 85, 90, 92, 94, 96, 98 or 99% to the sequence of *Pseudomonas putida* Gpo1 AlkB (database code: CAB54050.1), which polypeptide is capable of oxidizing alkanes. In one example, the AlkB monooxygenase may be an alkane-oxidizing oxidoreductase which functionally acts together with the *Pseudomonas putida* Gpo1 AlkG (CAB54052.1) and AlkT (CAB54063.1) polypeptides. For optimal supply of AlkB alkane hydroxylase with electrons, the cell expressing the monooxygenase together with auxiliary proteins that functionally interact with it, particularly AlkG and/or AlkT or respective variants thereof, from *Pseudomonas putida* Gpo1 AlkG (CAB54052.1) and AlkT (CAB54063.1) polypeptides may be used.

The ability of the second microorganism used in the process of the invention to oxidize substrates may be enhanced by the second microorganism expressing an alcohol dehydrogenase as an alternative to or additionally to the alkane hydroxylase. In particular, the term "alcohol dehydrogenase", as used herein, refers to an enzyme that oxidizes an aldehyde or ketone to the corresponding primary or secondary alcohol. Examples include the alcohol dehydrogenases of *Ralstonia eutropha* (ACB78191.1), *Lactobacillus brevis* (YP_795183.1), *Lactobacillus kefiri* (ACF95832.1), from horse liver, of *Paracoccus pantotrophus* (ACB78182.1) and *Sphingobium yanoikuyae* (EU427523.1), and also the respective variants thereof. The alkanes produced according to any aspect of the present invention may thus be readily used as a carbon source for the second microorganism to produce at least one respect oxidised alkane product. This method may allow for a purer source of alkane to be used to produce the oxidised products thus resulting in a more specific process of producing oxidised alkane products and less by-products being formed.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

### Clostridium kluyveri forming butyric acid from acetate and ethanol

For the biotransformation of ethanol and acetate to butyric acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 µl/l HCl (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂O, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 144 h to an OD₆₀₀ₙₘ >0.2.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 27 h to an OD₆₀₀ₙₘ >0.6. Then the cell suspension was centrifuged, washed with production buffer (pH 6.0; 8.32 g/L K-acetate, 0.5 g/l ethanol) and centrifuged again.

For the production culture, 200 ml of production buffer in a 500 ml bottle was inoculated with the washed cells from the main culture to an OD₆₀₀ₙₙ of 0.2. The culture was capped with a butyl rubber stopper and incubated for 71 h at 37°C and 100 rpm in an open water shaking bath. At the start and end of the culturing period, samples were taken. These were tested for optical density, pH and the different analytes (tested by NMR).

The results showed that in the production phase the amount of acetate decreased from 5.5 g/l to 5.0 g/l and the amount of ethanol decreased from 0.5 g/l to 0.0 g/l. Also, the concentration of butyric acid was increased from 0.05 g/l to 0.8 g/l and the concentration of hexanoic acid was increased from 0.005 g/l to 0.1 g/l.

### Example 2

### Clostridium kluyveri forming hexanoic acid from acetate and ethanol

For the biotransformation of ethanol and acetate to hexanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 µl/l HCl (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂O, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 144 h to an OD₆₀₀ₙₘ >0.2.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 27 h to an OD₆₀₀ₙₘ >0.6. Then the cell suspension was centrifuged, washed with production buffer (pH 6.0; 0.832 g/L K-acetate, 5.0 g/l ethanol) and centrifuged again.

For the production culture, 200 ml of production buffer in a 500 ml bottle was inoculated with the washed cells from the main culture to an OD₆₀₀ₙₙ of 0.2. The culture was capped with a butyl rubber stopper and incubated for 71h at 37°C and 100 rpm in an open water shaking bath. At the start and end of the culturing period, samples were taken. These were tested for optical density, pH and the different analytes (tested by NMR).

The results showed that in the production phase the amount of acetate decreased from 0.54 g/l to 0.03 g/l and the amount of ethanol decreased from 5.6 g/l to 4.9 g/l. Also, the concentration of butyric acid was increased from 0.05 g/l to 0.28 g/l and the concentration of hexanoic acid was increased from 0.03 g/l to 0.79 g/l.

### Example 3

### Clostridium kluyveri forming hexanoic acid from butyric acid and ethanol

For the biotransformation of ethanol and butyric acid to hexanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 pl/l HCl (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂O, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 144 h to an OD₆₀₀ₙₘ >0.3.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 25 h to an OD₆₀₀ₙₘ >0.4. Then the cell suspension was centrifuged, washed with production buffer (pH 6.16; 4.16 g/L K-acetate, 10.0 g/l ethanol) and centrifuged again.

For the production cultures, 200 ml of production buffer in a 500 ml bottle was inoculated with the washed cells from the main culture to an OD₆₀₀ₙₘ of 0.2. In a first culture, at the beginning 1.0 g/l butyric acid was added to the production buffer, in a second culture, no butyric acid was added to the production buffer. The cultures were capped with a butyl rubber stopper and incubated for 71 h at 37°C and 100 rpm in an open water shaking bath. At the start and end of the culturing period, samples were taken. These were tested for optical density, pH and the different analytes (tested by NMR).

The results showed that in the production phase of the butyric acid supplemented culture the amount of acetate decreased from 3.1 g/l to 1.1 g/l and the amount of ethanol decreased from 10.6 g/l to 7.5 g/l. Also, the concentration of butyric acid was increased from 1.2 g/l to 2.2 g/l and the concentration of hexanoic acid was increased from 0.04 g/l to 2.30 g/l.

In the production phase of the non-supplemented culture the amount of acetate decreased from 3.0 g/l to 1.3 g/l and the amount of ethanol decreased from 10.2 g/l to 8.2 g/l. Also, the concentration of butyric acid was increased from 0.1 g/l to 1.7 g/l and the concentration of hexanoic acid was increased from 0.01 g/l to 1.40 g/l.

### Example 4

### Clostridium kluyveri forming isohexanoic acid from isobutyric acid and ethanol

For the biotransformation of ethanol and isobutyric acid to isohexanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 µl/l HCl (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂O, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 144 h to an OD₆₀₀ₙₘ >0.3.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 25 h to an OD₆₀₀ₙₘ >0.4. Then the cell suspension was centrifuged, washed with production buffer (pH 6.16; 4.16 g/L K-acetate, 10.0 g/l ethanol) and centrifuged again.

For the production culture, 200 ml of production buffer in a 500 ml bottle was inoculated with the washed cells from the main culture to an OD₆₀₀ₙₘ of 0.2. At the beginning 1.0 g/l isobutyric acid was added to the production buffer. The culture was capped with a butyl rubber stopper and incubated for 71 h at 37°C and 100 rpm in an open water shaking bath. At the start and end of the culturing period, samples were taken. These were tested for optical density, pH and the different analytes (tested by NMR).

The results showed that in the production phase the amount of acetate decreased from 3.7 g/l to 1.0 g/l, the amount of ethanol decreased from 12.7 g/l to 7.8 g/l and the amount of isobutyric acid decreased from 1.30 g/l to 0.98 g/l. Also, the concentration of butyric acid was increased from 0.1 g/l to 1.6 g/l, the concentration of hexanoic acid was increased from 0.02 g/l to 1.80 g/l and the concentration of isohexanoic acid was increased from 0.00 g/l to 0.07 g/l.

### Example 5

### Clostridium kluyveri forming valeric acid and heptanoic acid from propionic acid and ethanol

For the biotransformation of ethanol and propionic acid to valeric acid and heptanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 µl/l HCl (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂O, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 119 h to an OD₆₀₀ₙₘ >0.2.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 21 h to an OD₆₀₀ₙₘ >0.4. Then the cell suspension was centrifuged, washed with production buffer (pH 6.0; 1.0 g/L propionic acid, 2.5 g/l ethanol) and centrifuged again.

For the production culture, 200 ml of production buffer in a 500 ml bottle was inoculated with the washed cells from the main culture to an OD₆₀₀ₙₙ of 0.2. The culture was capped with a butyl rubber stopper and incubated for 68 h at 37°C and 100 rpm in an open water shaking bath. At the start and end of the culturing period, samples were taken. These were tested for optical density, pH and the different analytes (tested by NMR).

The results showed that in the production phase the amount of propionic acid decreased from 1.08 g/l to 0.04 g/l and the amount of ethanol decreased from 2.5 g/l to 1.5 g/l. Also, the concentration of valeric acid was increased from 0.05 g/l to 0.95 g/l and the concentration of heptanoic acid was increased from 0.00 g/l to 0.29 g/l.

### References

Wadhawan J.D., et al., 2001, Pure and Applied Chemistry, 73(12):1947-1955, Steinbusch, 2011, Zhang, 2013, Van Eerten-Jansen, M. C. A. A, 2013, Ding H. et al, 2010, Barker H.A., 1949, Stadtman E.R., 1950, Bornstein B. T., et al., 1948, Byoung, S.J et al. 2013, Seedorf, H., et al., 2008, PNAS 105 (6): 2128-2133, 'Extraction Model' in Kieun C., et al., 2013, Mat-Jan et al., Bunch et al., 1997, Richard J. Heath et al., 1995, Chang et al., 1983, Abdel-Ahmid et al., 2001, Reed, 1981, Drake et al., 2006, Drake & Kusel, 2005, Wood, 1991, Drake et al., 2004, Scheps, D., Malca, H., Hoffmann, B., Nestl, B. M, and Hauer, B. (2011) Org. Biomol. Chem., 9, 6727, US20140051136, WO/2009/077461, WO 98/00558, WO 00/68407, WO2007/0275447 US2008/0057554

## Claims

1. A method of producing at least one alkane, the method comprising,
- producing at least one carboxylic acid from a carbon source using at least one microorganism, and
- performing Kolbe electrolysis on the carboxylic acid to produce the alkane,
wherein the alkane comprises at least 6 carbon atoms and the carboxylic acid comprises at least 4 carbon atoms.

2. The method according to claim 1, wherein the carbon source is selected from the group consisting of ethanol, acetate, propionate, butyrate, isobutyrate, valerate, hexanoate and combinations thereof and the microorganism is capable of producing the carboxylic acid using ethanol-carboxylate fermentation.

3. The method according to claim 2, wherein the microorganism is selected from the group consisting of *Clostridium kluyveri* and C. *Carboxidivorans.*

4. The method according to either claim 2 or 3, wherein the microorganism expresses at least one enzyme selected from the group consisting of alcohol dehydrogenase E₁, acetaldehyde dehydrogenase E₂, acetoacetyl-CoA thiolase E₃, 3-hydroxybutyryl-CoA dehydrogenase E₄, 3-hydroxybutyryl-CoA dehydratase E₅, butyryl-CoA dehydrogenase E₆, electron transfer flavoprotein subunit E₇, coenzyme A transferase E₈, acetate kinase E₉ and phosphotransacetylase E₁₀.

5. The method according to claim 4, wherein the microorganism expresses E₁, E₂, E₃, E₄, E₅, E₆, E₇, E₈, E₉ and E₁₀.

6. The method according to any one of claims 2 to 5, wherein the microorganism expresses hydrogenase maturation protein and/or electron transport complex protein.

7. The method according to any one of claims 2 to 6, wherein the microorganism is genetically modified and the genetically modified microorganism has increased expression relative to the wild type microorganism of at least one enzyme selected from the group consisting of E₁, E₂, E₃, E₄, E₅, E₆, E₇, E₈, E₉, E₁₀, hydrogenase maturation protein and/or electron transport complex protein.

8. The method according to claim 7, wherein the genetically modified microorganism has increased expression relative to the wild type microorganism of E₁, E₂, E₃, E₄, E₅, E₆, E₇, E₈, E₉ and E₁₀.

9. The method according to claim 1, wherein the carbon source is selected from the group consisting of glucose, sucrose, fructose, dextrose, lactose, xylose, pentose, polyol, hexose and synthesis gas, and the genetically modified organism is at least one acetogenic bacteria and/or hydrogen oxidising bacteria capable of producing at least one carboxylic acid from the carbon source.

10. The method according to claim 9, wherein the acetogenic bacteria is selected from the group consisting of *Clostridium autothenogenum* DSMZ 19630, *Clostridium ragsdahlei* ATCC no. BAA-622, *Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum* ATCC 33266, *Clostridium formicoaceticum, Clostridium butyricum, Laktobacillus delbrukii, Propionibacterium acidoprprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium Ijungdahlii, Clostridium* ATCC 29797 and *Clostridium carboxidivorans.*

11. The method according to claim 9, wherein the hydrogen oxidising bacteria is selected from the group consisting of *Achromobacter, Acidithiobacillus, Acidovorax, Alcaligenes, Anabena, , Aquifex, Arthrobacter, Azospirillum, Bacillus, Bradyrhizobium, Cupriavidus, Derxia, Helicobacter, Herbaspirillum, Hydrogenobacter, Hydrogenobaculum, Hydrogenophaga,, Hydrogenophilus, Hydrogenothermus, Hydrogenovibrio, Ideonella sp. O1, Kyrpidia, Metallosphaera, Methanobrevibacter, Myobacterium, Nocardia, Oligotropha, Para coccus, Pelomonas, Polaromonas, Pseudomonas, Pseudonocardia, Rhizobium, Rhodococcus, Rhodopseudomonas, Rhodospirillum, Streptomyces, Thiocapsa, Treponema,Variovorax,* and *Xanthobacter, Wautersia.*

12. The method according to any of the preceding claims, wherein the alkane is branched or unbranched.

13. The method according to any of the preceding claims, wherein the alkane is a symmetrical alkane.

14. The method according to claim 13, wherein the alkane and carboxylic acid are selected from the group consisting of:
(a) the alkane comprising 6 carbon atoms and the carboxylic acid comprising 4 carbon atoms;
(b) the alkane comprising 8 carbon atoms and the carboxylic acid comprising 5 carbon atoms;
(c) the alkane comprising 8 carbon atoms and a first carboxylic acid comprising 6 carbon atoms and a second carboxylic acid comprising 4 carbon atoms;
(d) the alkane comprising 10 carbon atoms and the carboxylic acid comprising 6 carbon atoms;
(e) the alkane comprising 10 carbon atoms and a first carboxylic acid comprising 5 carbon atoms and a second carboxylic acid comprising 7 carbon atoms;
(f) the alkane comprising 12 carbon atoms and the carboxylic acid comprising 7 carbon atoms;
(g) the alkane comprising 12 carbon atoms and a first carboxylic acid comprising 8 carbon atoms and a second carboxylic acid comprising 6 carbon atoms;
(h) the alkane comprising 12 carbon atoms and a first carboxylic acid comprising 9 carbon atoms and a second carboxylic acid comprising 5 carbon atoms;
(i) the alkane comprising 14 carbon atoms and the carboxylic acid comprising 8 carbon atoms; and
(j) the alkane comprising 14 carbon atoms and a first carboxylic acid comprising 6 carbon atoms and a second carboxylic acid comprising 8 carbon atoms.

15. The method according to claim 1 to 12, wherein the alkane is an asymmetrical alkane.

16. The method according to claim 15, wherein the alkane and carboxylic acid are selected from the group consisting of:
(a) alkane comprising 9 carbon atoms and a first carboxylic acid comprising 5 carbon atoms and a second carboxylic acid comprising 6 carbon atoms;
(b) alkane comprising 11 carbon atoms and a first carboxylic acid comprising 6 carbon atoms and a second carboxylic acid comprising 7 carbon atoms; and
(c) alkane comprising 13 carbon atoms and a first carboxylic acid comprising 7 carbon atoms and a second carboxylic acid comprising 8 carbon atoms.

17. The method according to claim 13, wherein the alkane and carboxylic acid are selected from the group consisting of:
(a) the alkane comprising 10 carbon atoms and the carboxylic acid comprising 6 carbon atoms;
(b) the alkane comprising 12 carbon atoms and the carboxylic acid comprising 7 carbon atoms; and
(c) the alkane comprising 14 carbon atoms and the carboxylic acid comprising 8 carbon atoms;

18. The method according to any of the preceding claims, wherein the method comprises a further step of isolating the alkane.

19. The method according to any of the preceding claims, wherein at least one olefin is produced after Kolbe electrolysis of the carboxylic acid.

20. The method according to any of the preceding claims, wherein the alkane is further subjected to catalytic reforming and/or isomerization to form gasoline or components of a gasoline composition.

21. An isolated alkane produced according to the method of any one of the claims 1 to 20.

22. A method of producing at least one oxidised alkane product, wherein the method comprises contacting the alkane of claim 21 with at least one second microorganism capable of oxidising the alkane to the respective oxidised alkane product, wherein the oxidised alkane product is selected from the group consisting of respective alcohols, carboxylic acids and dicarboxylic acids and the second microorganism is selected from the group consisting of *E*. *coli, Candida tropicalis, Yarrowia lipolytica* and *Pseudomonas putida.*
